# EUROPEAN PATENT APPLICATION

(11) **EP 4 279 576 A1**
(43) Date of publication of application: **22.11.2023**
(21) Application number: 21919836.3
(22) Date of filing: 01.10.2021
(51) Int. Cl.: C12N 1/04, C12N 1/12

(54) **COMPOSITION FOR FREEZE-PRESERVING MICROALGAE BELONGING TO FAMILY THRAUSTOCHYTRIACEAE AND METHOD FOR FREEZE-PRESERVING OF MICROALGAE BELONGING TO THRAUSTOCHYTRIACEAE USING SAME**

(30) Priority: 18.01.2021 KR 20210006814
(71) Applicant: CJ Cheiljedang Corporation, Seoul 04560 (KR)
(72) Inventor: JANG, Sung Hoon, Seoul 04560 (KR); SHIN, Won Sub, Seoul 04560 (KR); CHOI, Jung Woon, Seoul 04560 (KR); KANG, Hae Won, Seoul 04560 (KR); KIM, Ji Young, Seoul 04560 (KR); OK, Seung Han, Seoul 04560 (KR); JANG, Ho Sun, Seoul 04560 (KR); KIM, Jong Min, Seoul 04560 (KR); LEE, Jin Ho, Seoul 04560 (KR); KIM, Dae Cheol, Seoul 04560 (KR)
(74) Representative: Bates, Philip Ian
(86) International application number: PCT/KR2021/013515
(87) International publication number: WO 2022/154212

(57) **Abstract**

The present application relates to a composition for freeze-preserving microalgae belonging to the family Thraustochytriaceae and a method for freeze-preserving microalgae belonging to the family Thraustochytriaceae using same. By the composition for freeze-preserving microalgae belonging to the family Thraustochytriaceae according to an aspect and the method for freeze-preserving microalgae belonging to the family Thraustochytriaceae using same, the microalgae may be stored stably for a long period of time, and the costs for preservation of the microalgae may be reduced by shortening a process. In addition, according to a method for preparing freeze-dried biomass of microalgae belonging to the family Thraustochytriaceae using the composition, even during long-term storage at room temperature, freeze-dried biomass in the form of powder that can maintain bacterial activity and is easy for storage and transportation may be manufactured through a simple process.

## Description

### [TECHNICAL FIELD]

The present application relates to a composition for cryopreservation of microalgae of Thraustochytriaceae and a method for cryopreservation of microalgae of Thraustochytriaceae.

### [BACKGROUND ART]

Microalgae, which are phytoplankton, rank at the lowest level in the marine ecosystem food chain. Among them, microalgae belonging to Thraustochytriaceae are, unlike general microalgae, characterized as heterotrophs rather than autotrophs derived from photosynthesis, and play an important role in supplying omega-3 polyunsaturated fatty acids including docosahexaenoic acid and eicosapentaenoic acid to the marine ecosystem by producing and containing a high concentration of omega-3 polyunsaturated fatty acids including docosahexaenoic acid and eicosapentaenoic acid.

General microorganisms can be stored for a long time by cryopreservation or freeze-drying preservation methods. However, these general storage methods for microorganisms are not suitable for long-term effective storage of microalgae of Thraustochytriaceae. Accordingly, until now, such microalgae are preserved in the form of subculture. However, since the microalgae of Thraustochytriaceae are characterized as heterotrophs, they require more frequent passages than general microorganisms depending on the storage environment, thus making them vulnerable to contamination and resulting in high storage costs. Therefore, there is a need to develop a new method for long-term storage of microalgae of Thraustochytriaceae.

### [PRIOR ART DOCUMENTS]

### [PATENT DOCUMENTS]

(Patent Document 1) US Patent Publication US 2013/0089901 A1

### [DISCLOSURE]

### [TECHNICAL PROBLEM]

The present application is to provide a composition for cryopreservation of microalgae of Thraustochytriaceae, the composition comprising skim milk, sucrose, and sodium chloride.

The present application is to provide a method for cryopreservation of microalgae of Thraustochytriaceae using the composition for cryopreservation of microalgae of Thraustochytriaceae or a method of preparing a freeze-dried biomass of microalgae of Thraustochytriaceae.

The present application is to provide a freeze-dried biomass of microalgae of Thraustochytriaceae prepared by using the method for preparing the freeze-dried biomass of microalgae of Thraustochytriaceae.

The present application is to provide a use of the composition for cryopreservation of microalgae of Thraustochytriaceae, the composition comprising skim milk, sucrose, and sodium chloride, for the cryopreservation of microalgae of Thraustochytriaceae.

The present application is to provide a use of the composition for cryopreservation of microalgae of Thraustochytriaceae, the composition comprising skim milk, sucrose, and sodium chloride, for the preparation of the freeze-dried biomass of microalgae of Thraustochytriaceae.

### [TECHNICAL SOLUTION]

Each description and embodiment described in this application may also be applied to other descriptions and embodiments. That is, all combinations of the various elements described in this application fall within the scope of this application. In addition, it cannot be seen that the scope of the present application is limited by the detailed description described below. In addition, those skilled in the art would recognize, or be able to ascertain a plurality of equivalents with respect to particular aspects of the present application described in the present application by using conventional experiments only. Also, such equivalents are intended to be covered by this application.

An aspect provides a composition for cryopreservation of microalgae of Thraustochytriaceae, the composition comprising skim milk, sucrose, and sodium chloride.

The term "skim milk" refers to milk from which fat has been removed.

The term "Thraustochytriaceae" used herein is a microalgae belonging to the Thraustochytriales order, and the microalgae may include at least one selected from *Thraustochytrium* sp., *Schizochytrium* sp., *Aurantiochytrium* sp., *Thraustochytriidae* sp., *Japonochytrium* sp., *Monorhizochytrium* sp., *Sicyoidochytrium* sp., *Ulkenia* sp., *Parietichytrium* sp., *Botryochytrium* sp., *Hondaea* sp., and *Labyrinthulochytrium* sp. However, embodiments of the present disclosure are not limited thereto.

The composition may comprise 0.5 wt% to 20 wt% of skim milk based on the total weight of the composition. For example, the amount of the skim milk comprised in the composition may be from 0.5 wt% to 15 wt%, from 0.5 wt% to 10 wt%, from 0.5 wt% to 8 wt%, from 1 wt% to 20 wt%, from 1 wt% to 15 wt%, 1 wt% to 10 wt%, 1 wt% to 8 wt%, 2 wt% to 20 wt%, 2 wt% to 15 wt%, 2 wt% to 10 wt%, 2 wt% to 8 wt%, 3 wt% to 20 wt%, 3 wt% to 15 wt%, 3 wt% to 10 wt%, or 3 wt% to 8 wt%, based on the total weight of the composition.

The composition may comprise sucrose in an amount of 1 wt% to 20 wt% based on the total weight of the composition. For example, the amount of sucrose comprised in the composition may be from 1 wt% to 15 wt%, from 1 wt% to 12 wt%, from 2 wt% to 20 wt%, from 2 wt% to 15 wt%, from 2 wt% to 12 wt%, from 4 wt% to 20 wt%, from 4 wt% to 15 wt%, from 4 wt% to 12 wt%, from 6 wt% to 20 wt%, from 6 wt% to 15 wt%, from 6 wt% to 12 wt%, or from 6 wt% to 9 wt%, based on the total weight of the composition.

The composition may comprise sodium chloride in an amount of 0.1 wt% to 10 wt% based on the total weight of the composition. For example, the amount of sodium chloride comprised in the composition may be from 0.1 wt% to 9 wt%, 0.1 wt% to 8.5 wt%, 0.5 wt% to 10 wt%, 0.5 wt% to 9 wt%, 0.5 wt% to 8.5 wt%, 1 wt% to 10 wt%, 1 wt% to 9 wt%, 1 wt% to 8.5 wt%, 2 wt% to 10 wt%, 2 wt% to 9 wt%, 2 wt% to 8.5 wt%, 3 wt% to 10 wt%, 3 wt% to 9 wt%, 3 wt% to 8.5 wt%, 5 wt% to 8.5 wt%, or 6 wt% to 8.5 wt%, based on the total weight of the composition.

The composition may comprise skim milk and sucrose at a weight ratio of 1:0. 5 to 1:10. For example, the composition may comprise skim milk and sucrose at a weight ratio of 1:0.5 to 1:8, 1:0.5 to 1:5, 1:0.5 to 1:3, 1:1 to 1:10, 1:1 to 1:8, 1:1 to 1:5, 1:1 to 1:3, 1:1.2 to 1:10, 1:1.2 to 1:8, 1:1.2 to 1:5, or 1:1.2 to 1:3.

The composition may comprise skim milk and sodium chloride at a weight ratio of 1:0.5 to 1:10. For example, the composition may comprise skim milk and sodium chloride at a weight ratio of 1:0.5 to 1:8, 1:0.5 to 1:5, 1:0.5 to 1:3, 1:1 to 1:10, 1:1 to 1:8, 1:1 to 1:5, 1:1 to 1:3, 1:1.2 to 1:10, 1:1.2 to 1:8, 1:1.2 to 1:5, or 1:1.2 to 1:3.

The term "cryopreservation" used herein refers to preserving a material in a liquid state after freezing the same in a solid state, and may include "freeze-drying preservation". For example, the composition may be a composition for freeze-drying preservation of microalgae of Thraustochytriaceae. The term "freeze-drying" used herein refers to a drying method in which moisture in a sample is sublimed and removed therefrom by freezing the sample in the liquid state and leaving the same in reduced pressure. The freeze-drying can be used for a long-term preservation of microorganisms. However, an appropriate cryoprotectant should be used to prevent the damage of cells in the freeze-drying process.

The composition for cryopreservation of microalgae of Thraustochytriaceae comprising skim milk, sucrose, and sodium chloride can be used together with other cryoprotectants. For example, sodium chloride, dimethylsulfoxide (DMSO), dextran, sucrose, glycerol, mannitol, sorbitol, fructose, raffinose, serum albumin, and the like may be used in combination depending on the purpose, but embodiments of the present disclosure are not limited thereto.

Another aspect provides a method for cryopreservation of microalgae of Thraustochytriaceae, the method comprising: 1) culturing microalgae of Thraustochytriaceae in a medium containing the composition for cryopreservation of microalgae of Thraustochytriaceae comprising skim milk, sucrose and sodium chloride; 2) recovering the cultured product of process 1); and 3) freeze-drying the cultured product to produce biomass.

The composition for cryopreservation of the microalgae of Thraustochytriaceae is the same as described above.

The term "culture" used herein refers to growing the microalgae in an appropriately controlled environmental condition. The culturing process of the present application may be made according to suitable media and culture conditions known in the art. This culture process may be easily adjusted and used by those skilled in the art according to the selected microalgae.

Specifically, the culture of the microalgae of Thraustochytriaceae of the present application may be performed under heterotrophic conditions, but is not limited thereto.

The term "heterotrophic" is a nutritional method that depends on organic matter obtained from an energy source or a nutrient source outside the body, is a term distinguishable from autotrophic, and may be used interchangeably with the term "dark culture".

The process of culturing the microalgae of Thraustochytriaceae is not particularly limited thereto, but may be performed by a batch culture method, a continuous culture method, a fed-batch culture method, and the like, which are all known in the art. Any medium used for culturing the microalgae of the present application may be used without limitation as long as the same is a culture medium in which the microalgae of Thraustochytriaceae grows. Specifically, a conventional medium containing a carbon source, a nitrogen source, a phosphorus source, an inorganic compound, an amino acid and/or a vitamin which are suitable for the microalgae of the present application, may be used.

The carbon source comprised in the medium used in the culturing the microalgae of Thraustochytriaceae may be at least one selected from glucose, fructose, maltose, galactose, mannose, sucrose, arabinose, xylose and glycerol. However, any carbon source can be used as long as being used for culturing the microalgae.

The nitrogen source comprised in the medium used in the culturing the microalgae of Thraustochytriaceae may be i) at least one organic nitrogen source selected from yeast extract, beef extract, peptone and tryptone, or ii) at least one inorganic nitrogen source selected from ammonium acetate, ammonium nitrate, ammonium chloride, ammonium sulfate, sodium nitrate, urea, and monosodium glutamate (MSG). However, embodiments of the present disclosure are not limited thereto, and any nitrogen source that is used for culturing the microalgae can be used herein.

The medium used in the culturing the microalgae of Thraustochytriaceae maycomprise, as a phosphorus source, potassium dihydrogen phosphate, dipotassium hydrogen phosphate, a sodium-containing salt corresponding thereto, or a combination thereof. However, the phosphorus source is not limited thereto.

The culture conditions for culturing the microalgae of Thraustochytriaceae may be any culture condition as long as the microalgae of Thraustochytriaceae grows therein. For example, the culture may be performed while controlling temperature, pH, etc. in aerobic conditions.

Specifically, a basic compound (for example, sodium hydroxide, potassium hydroxide, or ammonia) or an acidic compound (for example, a phosphoric acid or a sulfuric acid) is used to adjust the pH to be at the appropriate level (for example, pH 5 to pH 9, specifically, pH 6 to 8) of the culture. However, embodiments of the present disclosure are not limited thereto.

In addition, in order to maintain the aerobic state of the culture, oxygen or oxygen-containing gas may be injected into the culture; or to maintain anaerobic and microaerobic conditions, gas may not be injected or nitrogen, hydrogen or carbon dioxide gas may be injected. However, embodiments of the present disclosure are not limited thereto.

In addition, the culture temperature may be maintained at about 20 °C to about 45 °C, or about 25 °C to about 40 °C, for about 10 hours to about 160 hours, about 10 hours to about 120 hours, about 10 hours to about 80 hours, about 10 hours to about 50 hours, or about 10 hours to about 40 hours. However, the culture conditions are not limited thereto. In addition, during the culture, an antifoaming agent such as a fatty acid polyglycol ester may be used to suppress the generation of bubbles, but embodiments of the present disclosure are not limited thereto.

The recovering the culture may be collecting the target culture by using a suitable method known in the art. For example, centrifugation, filtration, anion exchange chromatography, etc. may be used, but are not limited thereto.

The term "biomass" used herein refers to organisms such as plants, animals, and microorganisms that can be used as chemical energy, that is, an energy source of bioenergy, and may refer to, in terms of the ecological aspect, the weight or amount of energy of an organism which exist within a unit time and space. In addition, the biomass includes, but is not limited to, a compound secreted by a cell, and may contain an extracellular material as well as a cell and/or intracellular content. In the present application, the biomass may be a microalgae of Thraustochytriaceae itself, a culture thereof, or a product produced by culturing or fermenting the microalgae, or may be a concentrate of the biomass. However, the biomass is not limited thereto.

The "cultured product" of the microalgae of Thraustochytriaceae refers to a product produced by culturing the microalgae. Specifically, the cultured product may be a culture medium containing microalgae or a culture filtrate from which microalgae has been removed from the culture medium. However, the cultured product is not limited thereto. The cultured product of the microalgae of Thraustochytriaceae may be prepared by inoculating the microalgae in a microalgae culture medium and then proceeding the culture process according to a culture method known in the art.

The preparing of biomass by freeze-drying the cultured product may be performed according to a freeze-drying method known in the art. For example, the microalgae cultured product is recovered, put in a freeze-drying vial (FD vial), and connected to a freeze-drying device while maintaining a vacuum, and then water is removed therefrom while maintaining constant temperature and pressure conditions. The constant temperature condition may be, for example, a temperature of -50 °C or less, and the constant pressure condition may be a pressure of 0.133 mbar or less, but the temperature and pressure conditions are not limited thereto.

The method for cryopreservation of microalgae of Thraustochytriaceae may further include freezing the microalgae neither before nor after process 3). For example, the preliminary freezing process may not be performed before process 3). According to the method, microalgae may be preserved at low costs through a simple process by manufacturing freeze-dried biomass without including a preliminary freezing process.

Another aspect provides a method of preparing freeze-dried biomass of microalgae of Thraustochytriaceae, the method including: 1) culturing microalgae of Thraustochytriaceae in a medium containing the composition for cryopreservation of microalgae of Thraustochytriaceae comprising skim milk, sucrose, and sodium chloride; 2) recovering the cultured product of process 1); and 3) freeze-drying the cultured product to produce biomass.

The composition for cryopreservation of the microalgae of Thraustochytriaceae, culturing the microalgae of Thraustochytriaceae, recovering the cultured product, and preparing freeze-dried biomass are as described above.

The method of preparing the freeze-dried biomass of the microalgae of Thraustochytriaceae may further include freezing the microalgae neither before nor after process 3). For example, the preliminary freezing process may not be performed before process 3). According to the method, freeze-dried biomass may be prepared at low costs through a simple process by manufacturing freeze-dried biomass without including a preliminary freezing process.

Another aspect provides a freeze-dried biomass of microalgae of Thraustochytriaceae including: a) a composition for cryopreservation of microalgae of Thraustochytriaceae, the composition comprising skim milk, sucrose, and sodium chloride; and b) microalgae of Thraustochytriaceae, wherein the freeze-dried biomass is prepared by the method of preparing freeze-dried biomass of the microalgae of Thraustochytriaceae.

The composition for cryopreservation of the microalgae of Thraustochytriaceae and the method of preparing freeze-dried biomass of microalgae of Thraustochytriaceae are as described above.

The freeze-dried biomass may be stored at 15 °C to 25 °C for 12 weeks or more. For example, the freeze-dried biomass may be stored at room temperature for 3 months to 5 years, 3 months to 3 years, 3 months to 2 years, 3 months to 1 year, 3 months to 10 months, 3 months to 8 months, 3 months to 6 months.

The term "room temperature" used herein may refer to a temperature of about 15 °C to 25 °C, and may be used interchangeably with the term " ordinary temperature".

The term "storable" used herein may refer to the feature that cells can grow when the freeze-dried biomass is stored and then cultured in a medium again, or that the cell activity is maintained at 60% or more, 70% or more, 80% or more, or 90% or more, compared to live cells that are not freeze-dried.

The freeze-dried biomass may comprise 1.0 × 10⁷ to 1.0 × 10¹² live cells per 1 mL of biomass after storage at 15°C to 25 °C for at least 12 weeks. For example, after storage at 15 °C to 25 °C for at least 12 weeks, the freeze-dried biomass may comprise, per 1 mL, 1.0 × 10⁷ to 1.0 × 10¹¹, 1.0 × 10⁷ to 1.0 × 10¹⁰, 1.0 × 10⁸ to 1.0 × 10¹², 1.0 × 10⁸ to 1.0 × 10¹¹, 1.0 × 10⁸ to 1.0 × 10¹⁰, 1.0 × 10⁹ to 1.0 × 10¹², 1.0 × 10⁹ to 1.0 × 10¹¹, or 1.0 × 10⁹ to 1.0 × 10¹⁰ live cells.

The freeze-dried biomass may comprise 1.0 × 10⁷ to 1.0 × 10¹² CFU (colony forming unit)/mL live cells after storage at 15 °C to 25 °C for at least 12 weeks. For example, after storage at 15 °C to 25 °C for at least 12 weeks, the freeze-dried biomass may comprise 1.0 × 10⁷ to 1.0 × 10¹¹, 1.0 × 10⁷ to 1.0 × 10¹⁰, 1.0 × 10⁸ to 1.0 × 10¹², 1.0 × 10⁸ to 1.0 × 10¹¹, 1.0 × 10⁸ to 1.0 × 10¹⁰, 1.0 × 10⁹ to 1.0 × 10¹², 1.0 × 10⁹ to 1.0 × 10¹¹, or 1.0 × 10⁹ to 1.0 × 10¹⁰ CFU/mL live cells.

Another aspect provides a use of the composition for cryopreservation of microalgae of Thraustochytriaceae, the composition comprising skim milk, sucrose, and sodium chloride, for the cryopreservation of microalgae of Thraustochytriaceae.

Another aspect provides a use of the composition for cryopreservation of microalgae of Thraustochytriaceae, the composition comprising skim milk, sucrose, and sodium chloride, for the preparation of the freeze-dried biomass of microalgae of Thraustochytriaceae.

The composition for cryopreservation of the microalgae of Thraustochytriaceae, the method for cryopreservation of the microalgae of Thraustochytriaceae, and the method for preparing freeze-dried biomass of the microalgae of Thraustochytriaceae are as described above.

### [ADVANTAGEOUS EFFECTS]

According to the composition for cryopreservation of the microalgae of Thraustochytriaceae and the cryopreservation method using the same, the microalgae can be stably stored for a long period of time, and the microalgae preservation costs can be reduced by shortening the process. In addition, according to the method of preparing freeze-dried biomass of the microalgae of Thraustochytriaceae using the composition, the microbial activity is maintained even when storage for a long time at room temperature, and freeze-dried biomass in powder form, which is easy to store and transport, is prepared by a simple process.

### [BRIEF DESCRIPTION OF THE DRAWINGS]

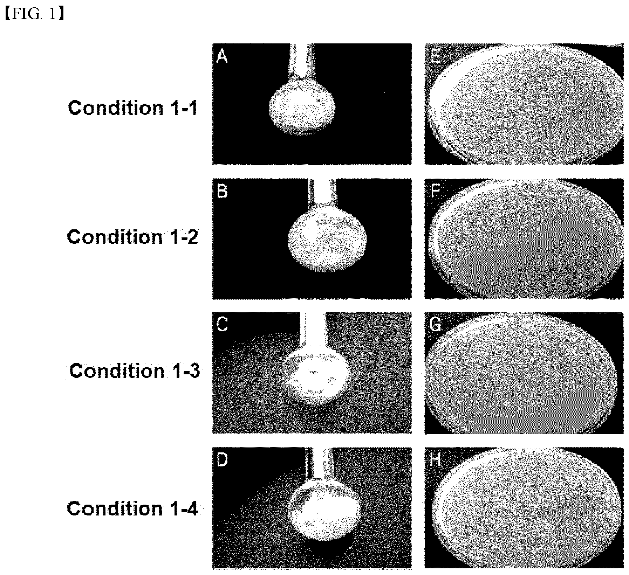

FIG. 1 shows images of freeze-dried vials (A to D) prepared according to an aspect, and images from which the formation of colonies after inoculation of the contents of the vials on an agar plate was confirmed (E to H) (AH: A shows an image of a freeze-dried vial prepared according to Condition 1-1, E shows an image from which it was confirmed that a colony had been formed after the contents of the vial of A was inoculated onto an agar plate, B shows an image of a freeze-dried vial prepared according to Condition 1-2, F shows an image from which it was confirmed that a colony had been formed after the contents of the vial of B was inoculated onto an agar plate, C shows an image of a freeze-dried vial prepared according to Condition 1-3, G shows an image from which it was confirmed that a colony had been formed after the contents of the vial of C was inoculated onto an agar plate, D shows an image of a freeze-dried vial prepared according to Condition 1-4, and H shows an image from which it was confirmed that a colony had been formed after the contents of the vial of D was inoculated onto an agar plate.).

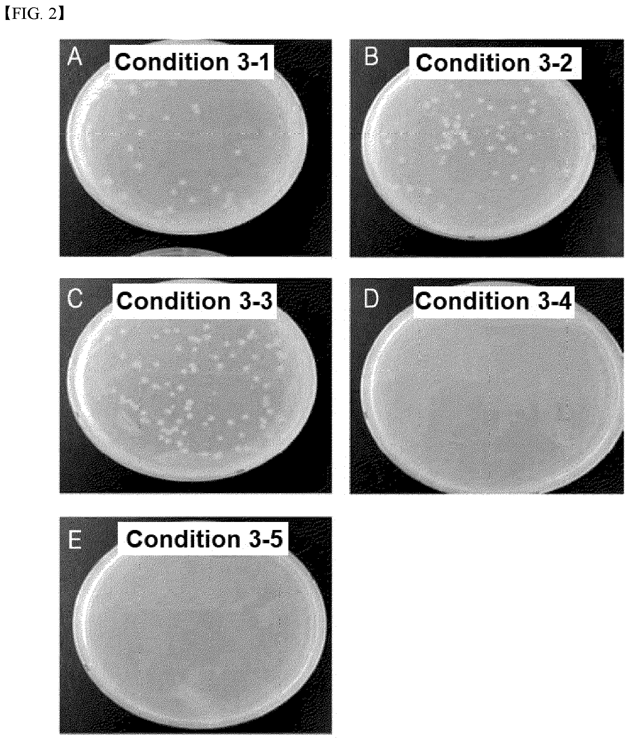

FIG. 2 shows the confirmation of a colony after freeze-dried biomass according to an aspect was inoculated on an agar plate (AE: A shows the confirmation of a colony after freeze-dried biomass according to Condition 3-1 was inoculated on an agar plate, B shows the confirmation of a colony after freeze-dried biomass according to Condition 3-2 was inoculated on an agar plate, C shows the confirmation of a colony after freeze-dried biomass according to Condition 3-3 was inoculated on an agar plate, D shows the confirmation of a colony after freeze-dried biomass according to Condition 3-4 was inoculated on an agar plate, E shows the confirmation of a colony after freeze-dried biomass according to Condition 3-5 was inoculated on an agar plate.).
FIG. 3 shows a growth curve graph in which absorbance is measured for each culture time after inoculation of a freeze-dried biomass according to an aspect in a culture flask.
FIG. 4 shows a growth curve graph in which absorbance is measured for each culture time after a freeze-dried biomass according to an aspect is stored at room temperature for 7 days and then inoculated in a culture flask.
FIG. 5 shows an image from which it was confirmed that a colony was formed after freeze-dried biomass according to an aspect was stored at room temperature for 12 weeks and then inoculated on an agar plate.

### [MODE FOR INVENTION]

Hereinafter, the present disclosure will be described in more detail through examples. However, these examples are provided to describe one or more embodiments for illustrative purposes, and the scope of the present disclosure is not limited to these examples.

### Example 1. Confirmation of cell viability according to freeze-drying conditions of microalgae

### Example 1-1. Production of Freeze-dried preservative

A total of 4 mL of freeze-dried preservative was prepared by mixing 0.8 mL of a 4% skim milk solution, 1.6 mL of a 20% sucrose solution, and 1.6 mL of a 20% sodium chloride solution, each dissolved in distilled water.

### Example 1-2. Freeze-dry microalgae of Thraustochytriaceae

Schizochytrium species microalgae CD01-5004 (Accession No.: KCTC14345BP) was inoculated on GYEP medium (10 g/L of glucose, 1 g/L of yeast extract, 1 g/L of peptone, 2 g/L of MgSO₄·7H₂O, 20 g/L of sea salt, 5.0 mg/L of H₃BO₃, 3.0 mg/L of MnCl₂, 0.2 mg/L of CuSO₄, 0.05 mg/L of NaMo₄·2H₂O, 0.05 mg/L of CoSO₄, and 0.7 mg/L of ZnSO₄·7H₂O), which was a medium in which microalgae of Thraustochytriaceae can grow, and incubated overnight in a 500 mL flask at 28 °C and at 180 rpm. The culture was recovered and centrifuged, and the supernatant was removed therefrom. The remaining resultant was suspended in the freeze-dried preservative prepared in Example 1-1 to prepare a microalgal biomass suspension. After the prepared biomass suspension was placed in an ampoule-type freeze-dried vial (FD vial), in the case of Conditions 1-1 to 1-3, pre-freezing was performed under the conditions as shown in Table 1, and a suspension that was not pre-frozen, was prepared as Condition 1-4.

**[Table 1]**

| Experimental group | Pre-freezing conditions | |
|---|---|---|
| Condition 1-1 | Normal freezing using freezing mixture | The biomass suspension was pre-frozen using a freezing mixture including 99% ethanol and dry ice. |
| Condition 1-2 | Gentle freezing | The biomass suspension was left at 4 °C for 60 minutes, then at -20 °C for 60 minutes, and then left at -80°C for 60 minutes to perform the pre-freezing under gentle conditions. |
| Condition 1-3 | Gental freezing + Drying at room temperature | After pre-freezing in the same conditions as in Condition 1-2, drying was performed at room temperature (25°C). |
| Condition 1-4 | No pre-freezing | - |

The freeze-dried vials of each condition were connected to a freeze-drying device, and freeze-dried under conditions of maintaining a temperature of -50 °C or less and a pressure of 0.133 mbar or less in a vacuum state. When a large number of ampoules are connected to the freeze-drying device, the pressure of the device is increased and thus the growth of freeze-dried cells is affected. Accordingly, one ampoule was connected, and then, after the pressure of the device was decreased to 0.133 mbar or less, the next ampoule was additionally connected thereto. After all the ampoules were connected to the device, the freeze-drying was performed for about 3 hours, and the ampoules that had been freeze-dried were sealed with a gas torch so that a vacuum could be maintained.

### Examples 1-3. Confirmation of viability of freeze-dried cells

In order to confirm the survival of the freeze-dried cells prepared in Example 1-2, a dry biomass sample in powder form was suspended in distilled water and spread on a GYEP agar plate, and then colony growth was visually checked.

As a result, as shown in FIG. 1, in the case of the freeze-dried biomass of Conditions 1-1 to 1-3 that had undergone the pre-freezing process, colonies were not grown or formed on the agar plate, and only in the case of Condition 1-4 that did not undergo the pre-freezing process, it was confirmed that the freeze-dried biomass enabled the growth and formation of colonies on agar plates.

### Example 2. Confirmation of the viability of freeze-dried cells according to the components of the freeze-dried preservative

In order to evaluate the freeze-drying preservation effect according to the components of the freeze-dried preservative, as shown in Table 2, for each condition, 4 mL of a freeze-dried preservative containing the corresponding components at the indicated final concentration was prepared.

**[Table 2]**

| Experimental group | Skim Milk (%) | Sucrose (%) | Trehalose (%) | Methano 1(%) | Peptone( %) | Glycerol (%) | Sodium Chloride (%) |
|---|---|---|---|---|---|---|---|
| Condition 2-1 | 10 | 10 | - | - | - | - | - |
| Condition 2-2 | 10 | - | 10 | - | - | - | - |
| Condition 2-3 | 10 | - | - | 10 | - | - | - |
| Condition 2-4 | - | - | 10 | - | 5 | - | - |
| Condition 2-5 | 10 | 4 | 4 | - | - | - | - |
| Condition 2-6 | 6 | 8 | - | - | - | - | 4 |
| Condition 2-7 | 10 | - | - | - | - | 10 | - |

The same method as used in Example 1-2 was used to prepare a biomass suspension, followed by freeze-drying without pre-freezing process, except that *Schizochytrium* sp. microalgae CD01-5004 (Accession No.: KCTC14345BP) was inoculated into GYEP medium containing 30 g/L of glucose, and the freeze-dried preservative prepared in the composition of Table 2 was used.

A dry biomass sample in powder form was suspended in distilled water and diluted so that the absorbance (Optical density: OD) at 680 nm was 0.1. The resultant solution was spread in an amount of 1 mL on a GYEP agar plate, and the number of colonies formed in each plate was counted. As a control, without suspending a part of the cell culture recovered before preparing the biomass suspension in a freeze-dried preservative, 1 mL of a solution diluted to an OD value of 0.1 at 680 nm was spread on a GYEP agar plate, and the number of colonies formed was counted. Since there are 1 × 10⁶ cells per colony, the number of viable cells per 1 mL of culture was calculated by multiplying the number of colonies formed for each experimental group by 10⁶, and the viability of each condition was calculated as a percentage of the control group.

**[Table 3]**

| Experimental group | Number of colonies formed | Number of live cells | Viability |
|---|---|---|---|
| Control | 1500 | 1.5 × 10⁹ | 100% |
| Condition 2-1 | 20 | 2.0 × 10⁷ | 1.33% |
| Condition 2-2 | 0 | 0 | 0% |
| Condition 2-3 | 0 | 0 | 0% |
| Condition 2-4 | 0 | 0 | 0% |
| Condition 2-5 | 2 | 2.0 × 10⁶ | 0.13% |
| Condition 2-6 | 250 | 2.5 × 10⁸ | 16.67% |
| Condition 2-7 | 0 | 0 | 0% |

As a result, as shown in Table 3, it was confirmed that colonies were formed within 72 hours after spreading on the plate under Conditions 2-1, 2-5, and 2-6. The freeze-dried preservatives used in these conditions commonly contained 6 to 10% of skim milk and 4 to 10% of sucrose, and in particular, in Condition 2-6 using a freeze-dried preservative containing skim milk, sucrose and sodium chloride, the viability was significantly high at 16.67%.

### Example 3. Confirmation of the growth degree of freeze-dried cells according to the ratio of the components of the freeze-dried preservative

### Example 3-1. Confirmation of colony formation on agar plates

In order to evaluate the freeze-dried preservation effect according to the concentration ratio of skim milk, sucrose and sodium chloride, which are the most effective preservative components derived in Example 2, as shown in Table 4 below, for each condition, 4 mL of a freeze-dried preservative containing the corresponding components at the indicated final concentration was prepared. After freeze-drying the microalgae of Thraustochytriaceae in the same manner as described in Example 2 using freeze-dried preservatives for each condition, the number of colonies formed was counted by suspending in distilled water and spreading the same on a GYEP agar plate. In the condition groups in which colonies were formed, colony formation was visually confirmed from 24 hours after spreading, and the presence or absence of cell survival and the degree of colony formation could be clearly distinguished from 40 hours.

**[Table 4]**

| Experimental group | Skim Milk (%) | Sucrose (%) | Sodium chloride (%) | Number of colonies |
|---|---|---|---|---|
| Condition 3-1 | 6 | 8 | 4 | 30 |
| Condition 3-2 | - | 10 | 5 | 32 |
| Condition 3-3 | 4 | 8 | 8 | 34 |
| Condition 3-4 | 10 | - | - | 0 |
| Condition 3-5 | 5 | - | 5 | 0 |

As a result, as shown in FIG. 2 and Table 4, colonies were not formed in Condition 3-4 using a preservative containing 10% skim milk alone, and Condition 3-5 using a preservative containing 5% skim milk and 5% sodium chloride, and, in the other conditions the formation of colonies was confirmed.

### Example 3-2. Confirmation of cell growth in culture flasks

In regard to the freeze-dried cells of Conditions 3-1 to 3-3 in which colony formation was confirmed in Example 3-1, the degree of cell growth in the culture flask was measured.

Specifically, a GYEP medium containing 30 g/L of glucose was placed in a 500 mL flask, and the freeze-dried biomass of Conditions 3-1 to 3-3 was inoculated thereto and cultured at 28°C and at 180 rpm. The culture of each condition was taken for each culture time, and the OD value was measured at 680 nm by using a spectrophotometer to confirm the growth degree of the cells.

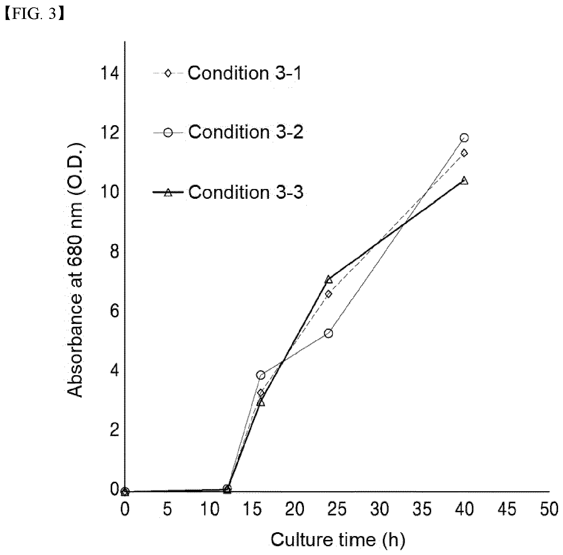

As a result, as shown in FIG. 3, the freeze-dried cells of Conditions 3-1 to 3-3 showed an increase in OD value through full-scale cell growth from about 12 hours after culture, and similar growth in all condition groups.

### Example 4. Confirmation of storage stability of freeze-dried samples according to the ratio of components of freeze-dried preservatives

After storing the freeze-dried biomass of Conditions 3-1 to 3-3 prepared in Example 3-1 at room temperature for 7 days in a freeze-dried vial, the biomass was cultured in a flask using the same method as described in Example 3-2, and the degree of growth of the cells was confirmed by measuring the absorbance for each culture time.

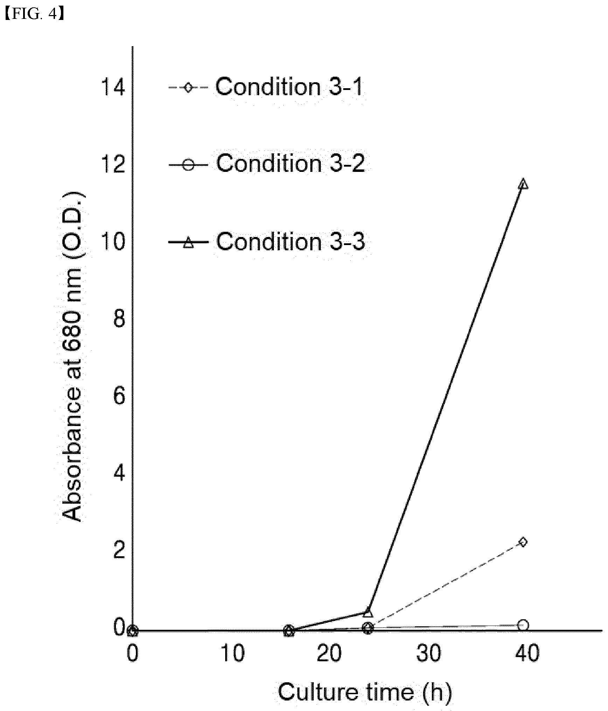

As a result, as shown in FIG. 4, it was confirmed that there was a difference in the degree of cell growth among conditions. Specifically, Condition 3-3 showed the fastest growth rate, condition 3-1 showed somewhat slow growth, and Condition 3-2 did not show cell growth until 40 hours after culture.

### Example 5. Long-term storage stability of freeze-dried biomass samples

In order to confirm the long-term storage stability of freeze-dried biomass samples, a freeze-dried preservative containing 4% of skim milk, 8% sucrose and 8% sodium chloride, which has the same freeze-dried preservative composition as in Condition 3-3 of Example 3-1, was prepared, and then, the microalgae of Thraustochytriaceae was freeze-dried in the same manner as described in Example 2. The prepared freeze-dried biomass was stored at room temperature for 12 weeks (84 days), then suspended in distilled water and spread on a GYEP agar plate to count the number of colonies formed.

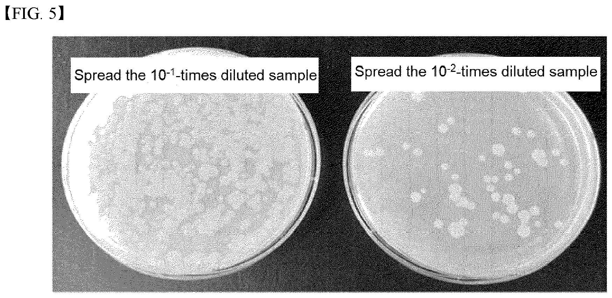

As a result, as shown in FIG. 5, about 47 colonies were formed in the 10⁻² -times diluted sample, and it was found that the number of viable cells per mL of culture was about 4.7 × 10⁹. Therefore, it was confirmed that the freeze-dried biomass produced using the freeze-dried preservative according to the present disclosure secured the number of viable cells in a certain amount or more even when stored for at least 12 weeks.

From the above description, those of ordinary skill in the art to which the present application pertains will be able to understand that the present application may be implemented in other specific forms without changing the technical spirit or essential characteristics thereof. In this regard, it should be understood that the embodiments described above are provided for illustrative purposes only in all respects and do not limit the scope of the present disclosure. The scope of the present application should be construed as including all changes or modifications derived from the meaning and scope of the claims described below and equivalent concepts thereof, rather than limited to the above detailed description.

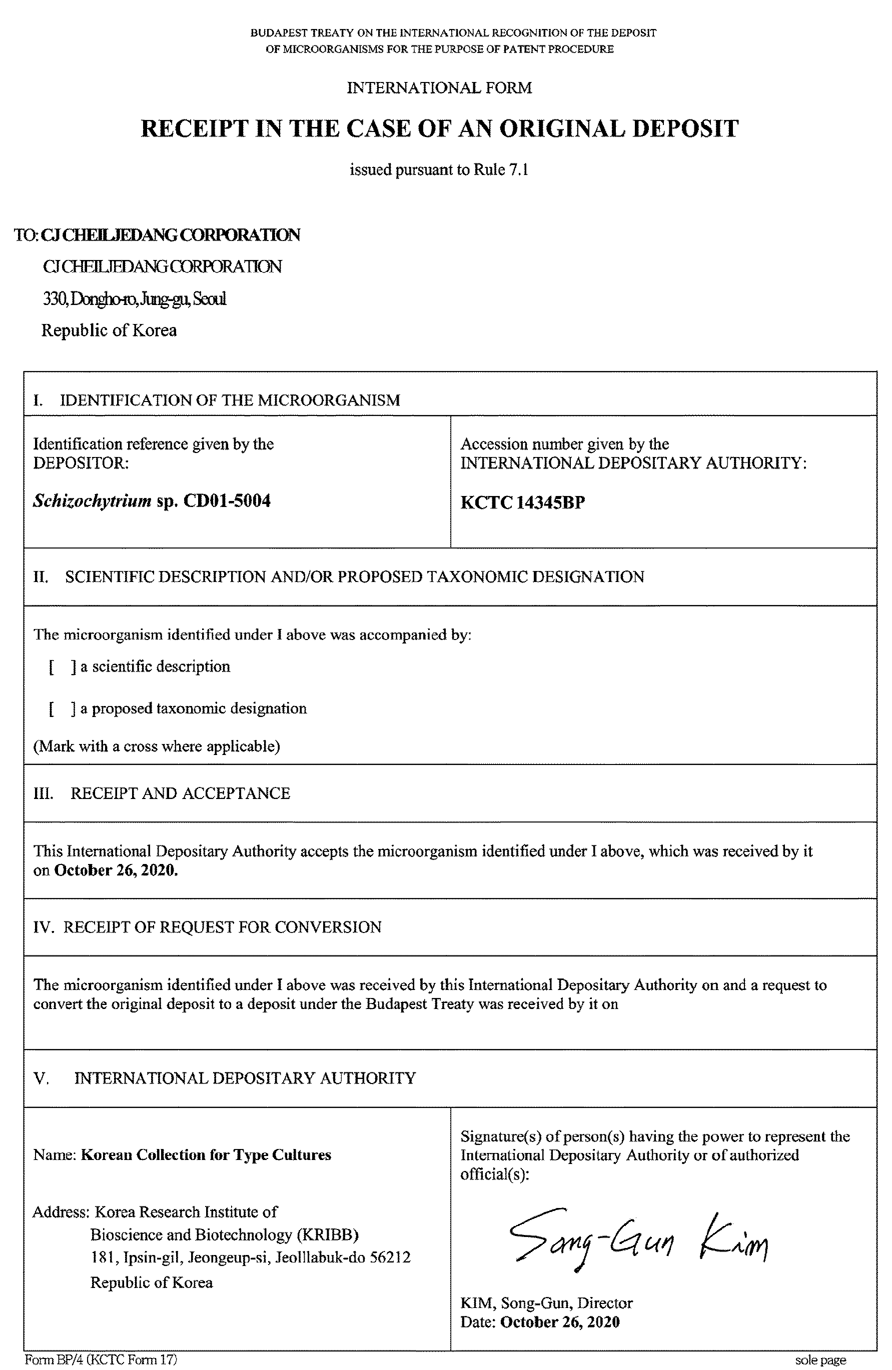
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330, Dongho-ro, Jung-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Schizochytrium sp. CD01-5004 | KCTC 14345BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on October 26, 2020.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: October 26, 2020

Form BP/4 (KCTC Form 17) sole page

## Claims

1. A composition for cryopreservation of microalgae of Thraustochytriaceae, the composition comprising: skim milk, sucrose, and sodium chloride.

2. The composition according to claim 1, wherein the composition comprises skim milk in an amount of 0.5 wt% to 20 wt% based on the total weight of the composition.

3. The composition according to claim 1, wherein the composition comprises sucrose in an amount of 1 wt% to 20 wt% based on the total weight of the composition.

4. The composition according to claim 1, wherein the composition comprises sodium chloride in an amount of 0.1 wt% to 10 wt% based on the total weight of the composition.

5. The composition according to claim 1, wherein the composition comprises skim milk and sucrose at a weight ratio of 1:0.5 to 1: 10.

6. The composition according to claim 1, wherein the composition comprises skim milk and sodium chloride at a weight ratio of 1:0. 5 to 1: 10.

7. The composition according to claim 1, wherein the microalgae of Thraustochytriaceae comprises at least one selected from *Thraustochytrium* sp., *Schizochytrium* sp., *Aurantiochytrium* sp., *Thraustochytriidae* sp., *Japonochytrium* sp., *Monorhizochytrium* sp., *Sicyoidochytrium* sp., *Ulkenia* sp., *Parietichytrium* sp., *Botryochytrium* sp., *Hondaea* sp., and *Labyrinthulochytrium* sp..

8. A method for cryopresevation of microalgae of Thraustochytriaceae, the method comprising:
1) culturing microalgae of Thraustochytriaceae in a medium comprising the composition for cryopreservation of microalgae of Thraustochytriaceae of claim 1;
2) recovering the cultured product of process 1); and
3) freeze-drying the recovered cultured product to prepare biomass.

9. The method according to claim 8, wherein freezing of the microalgae is performed neither before nor after process 3.

10. A method of preparing freeze-dried biomass of microalgae of Thraustochytriaceae, the method comprising:
1) culturing microalgae of Thraustochytriaceae in a medium comprising the composition for cryopreservation of microalgae of Thraustochytriaceae of claim 1;
2) recovering the cultured product of process 1); and
3) freeze-drying the recovered cultured product to prepare biomass.

11. The method according to claim 10, wherein freezing of the microalgae is performed neither before nor after process 3.

12. A freeze-dried biomass of microalgae of Thraustochytriaceae, the freeze-dried biomass comprising:
a) a composition for cryopreservation of microalgae of Thraustochytriaceae, the composition comprising: skim milk; sucrose; and sodium chloride; and
b) microalgae of Thraustochytriaceae,
wherein the freeze-dried biomass is produced by the method of preparing freeze-dried biomass of microalgae of Thraustochytriaceae of claim 10.

13. The freeze-dried biomass according to claim 12, wherein the freeze-dried biomass is able to be stored for at least 12 weeks at 15 °C to 25 °C.

14. The freeze-dried biomass according to claim 13, wherein the freeze-dried biomass comprises 1.0 × 10⁷ to 1.0 × 10¹² live cells per 1 mL of the freeze-dried biomass after storage at 15 °C to 25 °C for at least 12 weeks.
